# EUROPEAN PATENT APPLICATION

(11) **EP 4 803 041 A1**
(43) Date of publication of application: **09.09.2026**
(21) Application number: 24886190.8
(22) Date of filing: 29.10.2024
(51) Int. Cl.: A61F 2/24, G09B 23/30, B33Y 80/00, B33Y 50/00

(54) **METHOD FOR OPTIMIZING PERCUTANEOUS PULMONARY ARTERY VALVE INSERTION BY USING PATIENT-SPECIFIC MODEL AND IN VITRO SIMULATION CIRCULATION THROUGH 3D PRINTING**

(30) Priority: 30.10.2023 KR 20230147129; 21.10.2024 KR 20240143980
(71) Applicant: Seoul National University Hospital, Seoul 03080 (KR)
(72) Inventor: KIM, Yong Jin, Seoul 06601 (KR); LIM, Hong Gook, Seoul 06280 (KR); LEE, Whal, Seoul 06600 (KR); KIM, Gi Beom, Seoul 06291 (KR); SON, Kuk Hui, Incheon 22001 (KR)
(74) Representative: Pizzoli, Antonio Mario
(86) International application number: PCT/KR2024/016602
(87) International publication number: WO 2025/095516

(57) **Abstract**

The present invention relates to a method for optimizing percutaneous pulmonary valve implantation using a patient-specific model via 3D printing and in vitro mock circulation to find the optimal size and implantation position of a percutaneous pulmonary valve, thereby increasing the success rate of the percutaneous pulmonary valve implantation. The method according to the present invention includes the steps of: performing cardiac computed tomography (CT) to obtain a CT image; performing 3D modeling by segmenting the heart and blood vessels based on the CT image and then generating a 3D printing file format; and performing 3D printing using the 3D printing file to produce an outflow tract model 10 corresponding to a right ventricular outflow tract of a subject.

## Description

### [Technical Field]

The present invention relates to a method for optimizing percutaneous pulmonary valve implantation using a patient-specific model via 3D printing and in vitro mock circulation to find the optimal size and implantation position of a percutaneous pulmonary valve, thereby increasing the success rate of the percutaneous pulmonary valve implantation.

### [Background Art]

Generally, the heart consists of two atria and two ventricles, and includes four valves (an aortic valve, a pulmonary valve, a tricuspid valve, and a mitral valve) that serve to prevent backflow of blood between the respective parts.

Among these, the pulmonary valve, located between the right ventricle and the pulmonary artery of the heart, serves to prevent blood pumped from the right ventricle to the lungs from flowing back into the right ventricle, and pulmonary valve disease is a condition in which an abnormality occurs in the opening and closing of this valve.

Meanwhile, in the case of patients who have undergone pulmonary valve replacement surgery due to congenital right ventricular outflow tract malformation such as Tetralogy of Fallot, the occurrence of regurgitation due to the deterioration of valve function with age may cause the right ventricle to dilate or the valve to narrow; if severe stenosis occurs, the condition progresses to heart failure, which becomes highly critical.

To address these issues, open-chest and open-heart surgeries, in which the chest is opened and the heart is arrested to replace the pulmonary valve, have conventionally been performed. However, such reoperations involve significant pain and sequelae, and as the number of reoperations increases, the risk of complications also increases, leading to an urgent need for the development of alternative treatments to replace such surgical procedures.

As a treatment for this, there is percutaneous pulmonary valve implantation, which can improve pulmonary artery stenosis or regurgitation by inserting an artificial pulmonary valve through the femoral vein to expand the narrowed valve without making a chest incision.

However, for successful percutaneous pulmonary valve implantation, it was very important to find the optimal size and implantation position of a percutaneous pulmonary stent valve in right ventricular outflow tract diseases, which exhibit various sizes and shapes depending on each patient.

### [Disclosure]

### [Technical Problem]

The present invention is intended to solve the conventional problems, and when performing percutaneous pulmonary stent valve implantation, enables the procedure to be performed by varying the size and implantation position of the pulmonary stent valve according to the various sizes and shapes of the right ventricular outflow tract of each patient.

Accordingly, an object of the present invention is to perform segmentation of the heart and blood vessels based on CT or MR images of patients with complex cardiac malformations, create an STL file for 3D printing, and perform 3D printing using the created file to produce an outflow tract model that accurately models a complex anatomical structure.

Another object of the present invention is to perform patient-specific treatment using the produced outflow tract model through in vitro mock circulation.

### [Technical Solution]

To achieve the above objects, the present invention provides a method for optimizing percutaneous pulmonary valve implantation, comprising the steps of:
(S1) performing cardiac computed tomography (CT) to obtain a CT image;
(S2) performing 3D modeling by segmenting the heart and blood vessels based on the CT image and then generating a 3D printing file format; and
(S3) performing 3D printing using the 3D printing file to produce an outflow tract model corresponding to a right ventricular outflow tract of a subject.

### [Effects of the Invention]

According to the present invention as described above, it is possible to find the optimal size and implantation position of a percutaneous pulmonary valve by producing a patient's right ventricular outflow tract to mimic actual tissue using 3D printing and performing in vitro mock circulation, thereby increasing the success rate of future percutaneous pulmonary valve implantation and minimizing side effects.

### [Brief Description of the Drawings]

FIG. 1 is an image of various right ventricular outflow tracts of pulmonary valve disease according to an embodiment of the present invention.
FIG. 2 is an image of an artificial valve model according to an embodiment of the present invention.
FIG. 3 is an image of obtaining a CT image of a patient with pulmonary valve disease according to an embodiment of the present invention.
FIG. 4 is a 3D-modeled image based on a CT image according to an embodiment of the present invention.
FIG. 5 is an image of an in vitro mock circulation device and an artificial valve according to an embodiment of the present invention.

### [Mode for Carrying Out the Invention]

The features of a method for optimizing percutaneous pulmonary valve implantation using a patient-specific model via 3D printing and in vitro mock circulation according to the present invention will be understood through the embodiments described in detail below with reference to the accompanying drawings.

Meanwhile, the embodiments of the present invention may be implemented in various forms within the scope of the technical field to which the invention pertains, and are not particularly limited to the embodiments described herein.

Moreover, to clearly describe the present invention, repetitive descriptions of identical or similar components are omitted throughout the specification. Furthermore, parts irrelevant to the description are omitted from the drawings, and reference numerals for common components are omitted when describing such components.

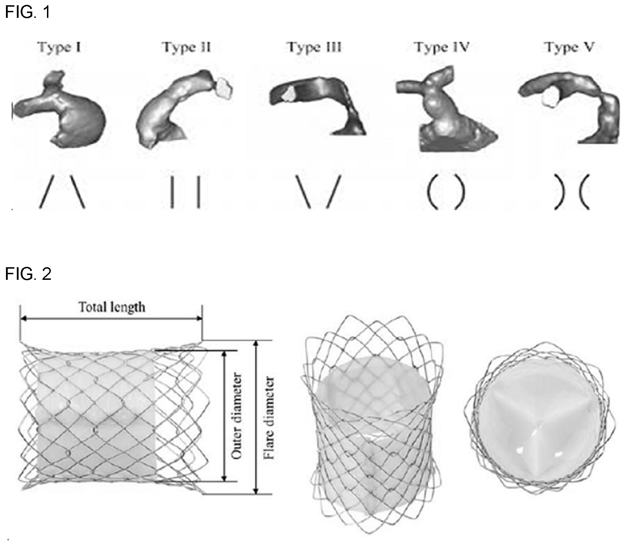

Hereinafter, a method for optimizing percutaneous pulmonary valve implantation according to an embodiment of the present invention will be described in detail with reference to FIGS. 1 to 5.

The method for optimizing percutaneous pulmonary valve implantation according to the present invention comprises the steps of: (S1) performing cardiac computed tomography (CT) to obtain a CT image; (S2) performing 3D modeling by segmenting the heart and blood vessels based on the CT image and then generating a 3D printing file format; and (S3) performing 3D printing using the 3D printing file to produce an outflow tract model 10 corresponding to a right ventricular outflow tract of a subject.

In addition, after step S3, the method further comprises the step of (S4) performing in vitro mock circulation by connecting the outflow tract model 10 to an in vitro mock circulation device 30.

Each of the above-described features will be described in detail below.

First, step S1 is for performing cardiac computed tomography (CT) of a subject to obtain a CT image.

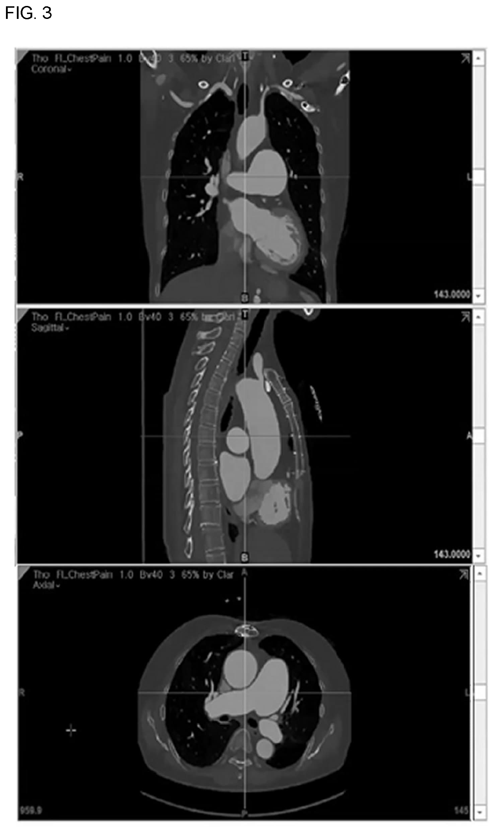

As shown in FIG. 3, this step allows for obtaining individual CT images of patients with complex cardiac malformations, which vary in size and shape, through cardiac computed tomography (CT). More specifically, while cardiac CT is used as a basis, images for generating a 3D printing file format can optionally be obtained through magnetic resonance imaging (MRI).

Furthermore, step S2 is for performing 3D modeling by segmenting the heart and blood vessels based on the CT image obtained in step S1, and then generating and providing a 3D printing file format.

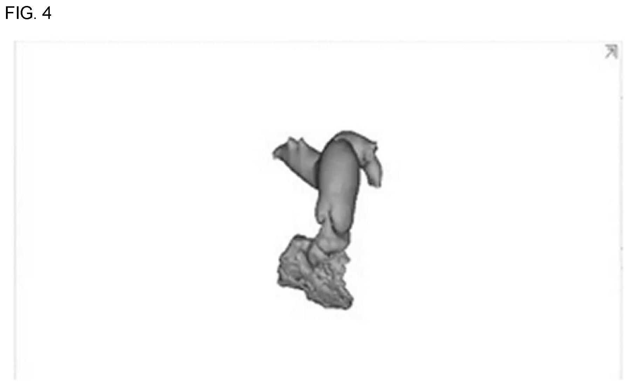

As shown in FIG. 4, this step involves utilizing a separate computer program, specifically software capable of performing free-form 3D modeling, to accurately perform 3D modeling of a specific portion, namely the complex anatomical structure of the right ventricular outflow tract, from the heart and blood vessels segmented from the CT image data of step S1, and then generating a 3D printing file format.

In this case, the 3D printing file format is a segmented 3D printing file format stored as a stereolithography file, and specifically, it is an STL file format widely used in CAD and 3D printing.

Step S3 is for producing an outflow tract model 10 corresponding to the right ventricular outflow tract of the subject by performing 3D printing using the 3D printing file.

In this step, the outflow tract model 10 is produced using a 3D printing device in the form of the 3D model from the 3D printing file generated in step S2 to mimic actual tissue, and accordingly, the outflow tract model 10 is composed of a material similar to actual tissue.

Meanwhile, as shown in FIG. 1, the right ventricular outflow tract is classified into Type 1 (Pyramidal), Type 2 (Straight), Type 3 (Reverse pyramidal), Type 4 (Convex), and Type 5 (Concave).

The pulmonary stent valve implanted into these right ventricular outflow tracts is an artificial valve 20, and the Pulsta pulmonary stent valve shown in FIG. 2 can be applied. This Pulsta pulmonary stent valve can be adapted to various shapes of the right ventricular outflow tract because it is relatively soft and compact.

Accordingly, when performing percutaneous pulmonary stent valve implantation using the artificial valve 20, the success rate of the procedure can be improved only by varying the size and implantation position of the pulmonary stent valve according to the various sizes and shapes of the right ventricular outflow tract of each patient.

Step S4 is for determining the optimal size and implantation position of the artificial valve 20 in the patient-specific outflow tract model 10 by performing in vitro mock circulation while connecting the outflow tract model 10 produced in step S3 to an in vitro mock circulation device 30.

In this step, artificial valves 20 of various sizes are inserted at specific positions into the outflow tract model 10 connected to the in vitro mock circulation device 30, and the specific positions correspond to a proximal part, a middle part, and a distal part of the main pulmonary artery.

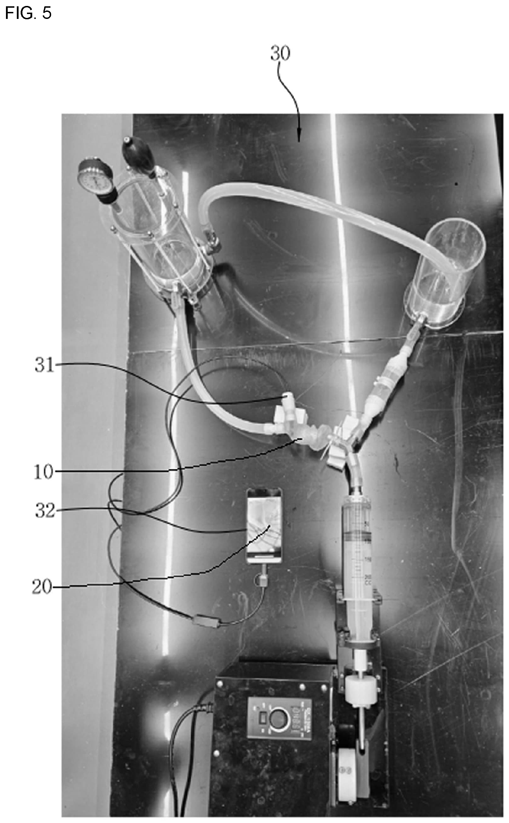

Meanwhile, as shown in FIG. 5, the in vitro mock circulation device 30 is configured to be connected to the outflow tract model 10 through a pipe and to generate a fluid flow similar to that of a human heart within the outflow tract model 10 by means of a pump.

In this case, after the artificial valve 20 is connected to the in vitro mock circulation device 30, in vitro mock circulation is performed to evaluate side effects such as displacement and migration of the artificial valve 20 within the outflow tract model 10 and to assess whether an insertion failure occurs. Subsequently, the optimal size and implantation position of the artificial valve 20 are determined in each patient-specific outflow tract model 10.

In this case, the in vitro mock circulation device 30 can determine the displacement and migration of the artificial valve 20 through an endoscope 31 capable of observing the inside of the outflow tract model 10, and the status can be monitored in real time by connecting a smartphone 32 to the endoscope 31.

Meanwhile, when the optimal size and implantation position of the artificial valve 20 are determined in the outflow tract model 10 through the in vitro mock circulation as described above, percutaneous pulmonary valve implantation is performed using the determined size and implantation position of the artificial valve 20.

Accordingly, when performing percutaneous pulmonary stent valve implantation, the procedure is performed by varying the size and implantation position of the artificial valve 20 according to the various sizes and shapes of the right ventricular outflow tract of each patient.

For example, the present invention can find the optimal size and implantation position of a percutaneous pulmonary valve by producing a patient's right ventricular outflow tract to mimic actual tissue using 3D printing and performing in vitro mock circulation, thereby increasing the success rate of future percutaneous pulmonary valve implantation and minimizing side effects.

As described above, although the preferred embodiments of the present invention have been described through the foregoing description and the accompanying drawings, the present invention is not particularly limited to the terms used to describe the present invention. The embodiments of the present invention can be variously changed or modified by those skilled in the art to which the present invention pertains without departing from the technical spirit of the invention, and the present invention is not limited to the embodiments in the detailed description and the claims.

## Claims

1. A method for optimizing percutaneous pulmonary valve implantation, comprising the steps of:
(S1) performing cardiac computed tomography (CT) to obtain a CT image;
(S2) performing 3D modeling by segmenting the heart and blood vessels based on the CT image and then generating a 3D printing file format; and
(S3) performing 3D printing using the 3D printing file to produce an outflow tract model corresponding to a right ventricular outflow tract of a subject.

2. The method for optimizing percutaneous pulmonary valve implantation according to claim 1, further comprising, after step S3, the step of (S4) performing in vitro mock circulation by connecting the outflow tract model to an in vitro mock circulation device.

3. The method for optimizing percutaneous pulmonary valve implantation according to claim 2, wherein step S4 comprises inserting artificial valves of various sizes at specific positions into the outflow tract model connected to the in vitro mock circulation device.

4. The method for optimizing percutaneous pulmonary valve implantation according to claim 3, wherein the specific positions at which the artificial valves are inserted correspond to a proximal part, a middle part, and a distal part of the main pulmonary artery.

5. The method for optimizing percutaneous pulmonary valve implantation according to claim 3, wherein the optimal size and implantation position of the artificial valve are determined by evaluating displacement and migration of the artificial valve within the outflow tract model.

6. The method for optimizing percutaneous pulmonary valve implantation according to claim 3, wherein the percutaneous pulmonary valve implantation is performed by varying the size and implantation position of the artificial valve according to various sizes and shapes of the right ventricular outflow tract of each patient.
